# EUROPEAN PATENT SPECIFICATION

(11) **EP 3 612 195 B1**
(45) Date of publication and mention of the grant of the patent: **09.04.2025**
(21) Application number: 18787287.4
(22) Date of filing: 17.04.2018
(51) Int. Cl.: A61K 35/34, A61K 38/18, A61P 21/00, C07K 14/47, C07K 14/485, C12N 5/077

(54) **A METHOD OF STIMULATING ASYMMETRIC DIVISION OF SATELLITE STEM CELLS**
VERFAHREN ZUR STIMULIERUNG DER ASYMMETRISCHEN TEILUNG VON SATELLITENSTAMMZELLEN
PROCÉDÉ DE STIMULATION DE DIVISION ASYMÉTRIQUE DE CELLULES SOUCHES SATELLITES

(30) Priority: 21.04.2017 US 201762488475 P
(43) Date of publication of application: 26.02.2020
(73) Proprietor: Ottawa Hospital Research Institute, Ottawa, ON K1N 6N5 (CA)
(72) Inventor: RUDNICKI, Michael, Ottawa ON K1B 3J3 (CA); WANG, Yu, Xin, Sunnyside, CA 94086 (US)
(74) Representative: Elend, Almut Susanne
(86) International application number: PCT/US2018/027920
(87) International publication number: WO 2018/195046

(56) References cited:
- US-A1- 2017 087 188
- ODA KANAE ET AL: "A comprehensive pathway map of epidermal growth factor receptor signaling", MOLECULAR SYSTEMS BIOLOGY, THE MACMILLAN BUILDING, LONDON, GB, vol. 1, 1 January 2005 (2005-01-01), XP002436396, ISSN: 1744-4292, DOI: 10.1038/MSB4100014
- YU XIN WANG ET AL: "EGFR-Aurka Signaling Rescues Polarity and Regeneration Defects in Dystrophin-Deficient Muscle Stem Cells by Increasing Asymmetric Divisions", CELL STEM CELL, vol. 24, no. 3, 1 March 2019 (2019-03-01), AMSTERDAM, NL, pages 419 - 432.e6, XP055754487, ISSN: 1934-5909, DOI: 10.1016/j.stem.2019.01.002
- WANG, YX: "Molecular Regulation of Muscle Stem Cell Self-Renewal", DOCTORAL THESIS UNIVERSITY OF OTTAWA, 2016, XP055544127, Retrieved from the Internet <URL:http://www.ruor.uottawa.ca/bitstream/10393/35207/1/Wang_Yu_Xin_2016_thesis.pdf> [retrieved on 20180606]

## Description

### FIELD

The present disclosure relates to compositions and methods for stimulating asymmetric division of satellite stem cells.

### BACKGROUND

The following paragraphs are not an admission that anything discussed in them is prior art or part of the knowledge of persons skilled in the art.

Muscular dystrophy is a group of muscle diseases that results in increasing weakening and breakdown of skeletal muscles over time. Different types of muscular dystrophy are due to mutations in different genes that are involved in making muscle proteins. Some variants of muscular dystrophy are due to a loss of, or a reduced production of, dystrophin. A major function of dystrophin is to stabilize the membrane of myofibers. The loss of dystrophin leads to a weakening of the myofiber membrane and predisposes patients to muscle damage.

### INTRODUCTION

Dysregulation of muscle stem cell function is directly associated with reduced regeneration in aging and with Duchenne muscular dystrophy (DMD), a progressive and severe myopathy caused by loss-of-function mutations in dystrophin. Human dystrophin protein may have a sequence as set out in Uniprot entry number P11532. Activation of muscle satellite cells to proliferate, differentiate, and fuse is necessary for repair of damaged muscle fibers or formation of new muscle fibers. However, loss of dystrophin in satellite cells leads to a loss of cell polarity, which impairs asymmetric cell divisions and impairs generation of committed myogenic progenitors. The dystrophin-deficient satellite stem cells also display prolonged cell cycle kinetics, and abnormal division patterns.

Other genetic diseases, including those with mutations in genes which encode components of the dystrophin-associated glycoprotein complex (DGC) or are involved in their processing, for example glycosylation, may also result in an failure to assemble the DGC in the membrane, resulting in an inability for dystrophin to be stabilized at the membrane.

Dystrophin-deficient satellite stem cells generate myogenic progenitors, which are needed for muscle regeneration, at a reduced rate in comparison to normal satellite stem cells. Similarly, satellite cells having an inability, or a reduced ability, to assemble a functional dystrophin-associated glycoprotein complex (DGC), resulting in an inability, or reduced ability, to establish cell polarity also generate myogenic progenitors at a reduced rate. The regulation of satellite stem cell asymmetric division is a control point that impacts the efficiency of the muscle regenerative program. There remains a need for compounds or methods that stimulate asymmetric division of satellite stem cells in a patient suffering from such a disease or disorder. One example of such a disease or disorder is muscular dystrophy, such as Duchenne muscular dystrophy. Such compounds or methods may improve or rescue satellite cell function in the patient, and thereby reduce the severity of a symptom of the disease or disorder. In some cases, such compounds or methods may ameliorate or reverse one or more symptoms of the disease or disorder.

The inventors have determined that polarized propagation of the epidermal growth factor receptor (EGFR) pathway is a determinant of asymmetric satellite stem cell divisions. Epidermal growth factor receptor may have a sequence as set out in Uniprot entry number P00533. They have determined that the EGFR polarity pathway acts independently of dystrophin, and stimulation of the EGFR pathway with recombinant EGF is able to stimulate Aurka kinase A to establish polarity, resulting in an increased proportion of asymmetric divisions in dystrophin-deficient satellite cells. Human Aurka kinase A may have a sequence as set out in Uniprot entry number O14965.

The invention provided herein is defined according to the appended claims.

Described herein is a method for stimulating asymmetric division of at least some satellite stem cells in a patient suffering from a disease or disorder characterized by satellite cells having an inability, or a reduced ability, to assemble a functional dystrophin-associated glycoprotein complex (DGC) that results in an inability, or reduced ability, to establish cell polarity. The method includes administering to the patient a sufficient amount of an epidermal growth factor receptor (EGFR) pathway activator to stimulate asymmetric division of at least some satellite stem cells. The invention provides an EGFR pathway activator for use in the treatment of a disease or disorder characterized by satellite cells having an inability, or a reduced ability, to assemble a functional dystrophin-associated glycoprotein complex (DGC) that results in an inability, or reduced ability, to establish cell polarity, wherein the EGFR pathway activator is formulated for administration in an amount sufficient to stimulate asymmetric division of at least some satellite stem cells. Also described is the use of an EGFR pathway activator for: stimulating asymmetric division of at least some satellite stem cells in a patient suffering from a disease or disorder characterized by satellite cells having an inability, or a reduced ability, to assemble a functional dystrophin-associated glycoprotein complex (DGC) that results in an inability, or reduced ability, to establish cell polarity, or in the manufacture of a medicament for stimulating asymmetric division of at least some satellite stem cells in a patient suffering from a disease or disorder characterized by satellite cells having an inability, or a reduced ability, to assemble a functional dystrophin-associated glycoprotein complex (DGC) that results in an inability, or reduced ability, to establish cell polarity.

When the disease or disorder is muscular dystrophy, the satellite stem cells are characterized by expression of paired box 7 (PAX7) protein and EGFR protein, and by a lack of expression of myogenic factor 5 (Myf5) protein, and MyoD protein. Human PAX7 protein may have a sequence as set out in Uniprot entry number P23759. Human Myf5 protein may have a sequence as set out in Uniprot entry number P13349. Human MyoD protein may have a sequence as set out in P15172.

Stimulating asymmetric division of at least some satellite stem cells may improve or rescue satellite cell function, improve or restore the proportion of asymmetric divisions of satellite stem cells, or any combination thereof.

Stimulating asymmetric division of at least some satellite stem cells in the patient may reduce the severity of one or more symptoms of the disease or disorder in the patient.

### DESCRIPTION OF THE FIGURES

**Figures 1** **and** **2** **are depictions of how EGFR Signaling Regulates Asymmetric Satellite Stem Cell Divisions**
**Figure 1(A)** shows the number of asymmetric satellite stem cell divisions per myofiber at 42h of culture in the presence of DMSO control (vehicle) or Lapatinib normalized to the vehicle control.
**Figure 1(B)** shows the number of YFP- satellite stem cells per myofiber at 42h of culture in the presence of DMSO control (vehicle) or Lapatinib normalized to the vehicle control.
**Figure 1(C)** shows the number of asymmetric satellite stem cell divisions per myofiber at 42h of culture after transfection with scrambled control siRNA (siSCR) or siRNA against *EGFR* (si*EGFR*) normalized to the siSCR control.
**Figure 1(D)** shows the relative number of YFP- satellite stem cells per myofiber at 42h of culture after transfection with scrambled control siRNA (siSCR) or siRNA against *EGFR* (si*EGFR*) normalized to the siSCR control. The error bars for FIG. 1 represent means ± SEM; p-values: *=< 0.05; **=< 0.01; ***=< 0.005.
**Figure 2(A)** shows the elative number of asymmetric satellite stem cell divisions per myofiber at 42h of culture in the presence of 1% BSA in PBS control (vehicle) or recombinant EGF.
**Figure 2(B)** shows the relative number of apicobasally oriented mitotic satellite cell divisions at 36h of culture in the presence of 1% BSA in PBS control (vehicle) or recombinant EGF.
**Figure 2(C)** shows the planar (upper) and apicobasal (lower) orientation of p-Aurk centrosome staining in satellite cells on single myofibers at 36h of culture. The host myofiber of the satellite cell is outlined with the dashed line. The error bars for FIG. 2 represent means ± SEM; p-values: *=< 0.05; **=< 0.01; ***=< 0.005.

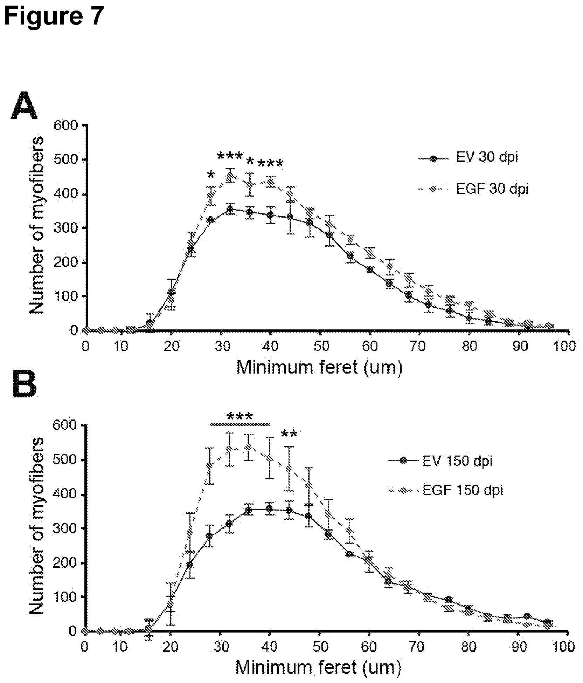

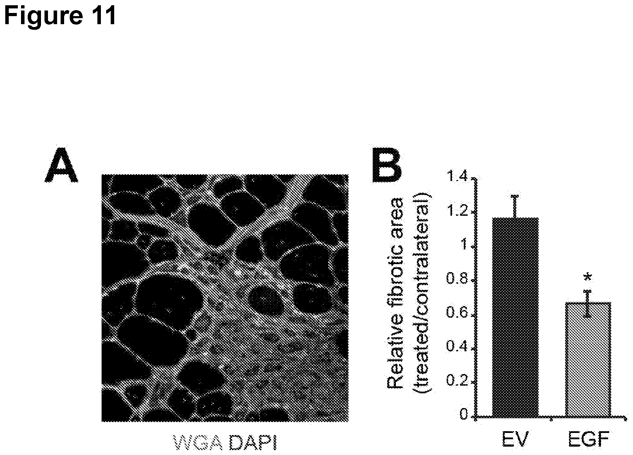

**Figures 3-13** show how EGF Stimulation Rescues Polarity Deficits in *mdx* Satellite Cells
**Figure 3(A)** shows the signaling status of p-EGFR (white) in Pax7+ (gray) satellite cells on EDL myofibers after 1h culture in Ham's F10 media with 1% BSA in PBS control (vehicle) or 100ng/mL of recombinant EGF. DNA is stained with DAPI (dark grey).
**Figure 3(B)** shows the quantification of abnormal, versus planar, and apicobasal orientated mitotic spindles in satellite cells on *mdx* myofibers at 36h of culture in the presence of 1% BSA in PBS control (vehicle) or recombinant EGF. The error bars for FIG.3 represent means ± SEM; p-values: *=< 0.05; **=< 0.01; ***=< 0.005.
**Figure 4(A)** shows the quantification of asymmetric divisions relative to total satellite stem cell divisions in WT and *mdx* myofibers at 42h of culture in the presence of 1% BSA in PBS control (vehicle) or recombinant EGF.
**Figure 4(B)** shows the quantification of asymmetric divisions per myofiber in WT and *mdx* myofibers at 42h of culture in the presence of 1% BSA in PBS control (vehicle) or recombinant EGF.
**Figure 4(C)** shows the quantification of Myog-expressing cells per *mdx* myofiber at 72h of culture in the presence of 1% BSA in PBS control (vehicle) or recombinant EGF.
**Figure 4(D)** shows the quantification of total myogenic cells (Pax7-expressing or Myog-expressing cells) per *mdx* myofiber at 72h of culture in the presence of 1% BSA in PBS control (vehicle) or recombinant EGF. The error bars for FIG.4 represent means ± SEM; p-values: *=< 0.05; **=< 0.01; ***=< 0.005.
**Figure 5(A)** shows a schematic overview of cardiotoxin-induced injury and treatment with recombinant EGF protein in *mdx* mice.
**Figure 5(B)** shows a quantification of Pax7+ cells on frozen sections from regenerating *mdx* TA muscles 10 days post cardiotoxin-induced injury and treated with saline (vehicle), or recombinant EGF protein.
**Figure 5(C)** shows a quantification of Myog+ cells on frozen sections from regenerating *mdx* TA muscles 10 days post cardiotoxin-induced injury and treated with saline (vehicle), or recombinant EGF protein.
**Figure 5(D)** shows a distribution of minimum Feret of myofibers from regenerating *mdx* TA muscles 10 days post cardiotoxin-induced injury and treated with saline (vehicle), or recombinant EGF protein. The error bars on FIG. 5 represent means ± SEM; p-values: *=< 0.05; **=< 0.01; ***=< 0.005.
**Figure 6** A) shows a schematic overview of electroporation of EGF expression vector in young *mdx* mice and in situ force measurements and histology at 30 days and 150 days post injury.
**Figure 6(B)** shows the relative muscle mass of TA muscles of *mdx* mice electroporated with empty vector (EV) or EGF expression vector (EGF) normalized to their contralateral legs at 30 days and at 150 days.
**Figure 6(C)** shows a cross sectional area of TA muscles of *mdx* mice electroporated with empty vector (EV) or EGF expression vector (EGF) normalized to their contralateral legs at 30 days and at 150 days.
**Figure 6(D)** shows the telative expression of Myog-expressing cells per mm² myofiber of *mdx* mice electroporated with empty vector (EV) or EGF expression vector (EGF) normalized to their contralateral legs at 30 days and at 150 days.
**Figure 6(E)** shows a quantification of myofibers in TA muscle of *mdx* mice electroporated with empty vector (EV) or EGF expression vector (EGF) compared to their contralateral legs. The error bars for FIG. 6 represent means ± SEM; p-values: *=< 0.05; **=< 0.01; ***=< 0.005.
**Figure 7(A)** shows the distribution of minimum Feret of myofibers from regenerating mdx TA muscles 30 days post cardiotoxin-induced injury (dpi) and treated with empty vector (EV) or EGF expression vector (EGF) compared to their contralateral legs.
**Figure 7(B)** shows the distribution of minimum Feret of myofibers from regenerating mdx TA muscles 150 days post cardiotoxin-induced injury and treated with empty vector (EV) or EGF expression vector (EGF) compared to their contralateral legs. The error bars on FIG. 7 represent means ± SEM; p-values: *=< 0.05; **=< 0.01; ***=< 0.005.
**Figure 8(A)** shows the maximum tetanic force (mN) of myofibers from regenerating *mdx* TA muscles 30 dpi and 150 dpi, treated with empty vector (EV) or EGF expression vector (EGF).
**Figure 8(B)** shows the maximum specific force (mN/cm²) of myofibers from regenerating *mdx* TA muscles 30 dpi and 150 dpi, treated with empty vector (EV) or EGF expression vector (EGF). The error bars for FIG. 8 represent means ± SEM; p-values: *=< 0.05; **=< 0.01; ***=< 0.005.
**Figure 9(A)** shows a schematic overview of electroporation of EGF expression vector in young *mdx* mice and harvesting and force measurements time points.
**Figure 9(B)** shows Relative muscle mass of TA muscles of *mdx* mice electroporated with empty vector (EV) or EGF expression vector (EGF) at 24 weeks normalized to their contralateral legs.
**Figure 9(C)** shows the relative average minimum Feret of myofibers from regenerating TA muscles of *mdx* mice electroporated with empty vector (EV) or EGF expression vector (EGF) at 24 weeks normalized to their contralateral legs.
**Figure 9(D)** shows the number of myofibers from regenerating TA muscles of *mdx* mice electroporated with empty vector (EV) or EGF expression vector (EGF) at 24 weeks in contralateral (contra) legs and treated legs. Error bars for FIG. 9 represent means ± SEM; p-values: *=< 0.05; **=< 0.01; ***=< 0.005.
**Figure 10(A)** shows Immunostaining for Myh3+ (gery) newly formed myofibers, Myh4+ (black) type-IIb fast twitch myofibers, and necrotic myofibers infiltrated with mouse immunoglobulin G (msIgG+, white) on frozen sections from electroporated TA muscles of *mdx* mice. DNA is stained with DAPI (dark grey).
**Figure 10(B)** shows the regenerative index of TA muscle of *mdx* mice electroporated with empty vector (EV) or EGF expression vector (EGF) compared to their contralateral legs. Error bars represent means ± SEM; p-values: *=< 0.05; **=< 0.01; ***=< 0.005.
**Figure 11(A)** shows a representative image of wheat-germ agglutinin (WGA) stained (grey) fibrotic deposits on frozen sections from electroporated TA muscles of *mdx* mice. DNA is stained with DAPI (dark grey).
**Figure 11(B)** shows the quantification of WGA+ fibrotic areas in TA muscle of *mdx* mice electroporated with empty vector (EV) or EGF expression vector (EGF) normalized to their contralateral legs. The error bars bars for FIG> 11 represent means ± SEM; p-values: *=< 0.05; **=< 0.01; ***=< 0.005.
**Figure 12(A)** shows immunoblotting analysis of p-EGFR in serum-starved myoblasts that were refed with growth media, growth media supplemented with recombinant EGF, growth media supplemented with PTP1b inhibitor, or growth media supplemented with recombinant EGF and PTP1b inhibitor for 1h.
**Figure 12(B)** shows grey value quantification of p-EGFR in myoblasts treated with recombinant EGF and PTP1b inhibitor.
**Figure 12(C)** shows a quantification of asymmetric divisions relative to total satellite stem cell divisions in WT myofibers at 42h of culture in the presence of vehicle control, recombinant EGF, PTP1b inhibitor, or both recombinant EGF and PTP1b inhibitor.
**Figure 12(D)** shows a quantification of asymmetric divisions relative to total satellite stem cell divisions in mdx myofibers at 42h of culture in the presence of vehicle control, recombinant EGF, PTP1b inhibitor, or both recombinant EGF and PTP1b inhibitor. The error bars for FIG. 12 represent means ± SEM; p-values: *=< 0.05; **=< 0.01; ***=< 0.005.
**Figure 13(A)** shows a schematic overview of cardiotoxin-induced injury and treatment with PTP1b inhibitor in mdx mice.
**Figure 13(B)** shows immunostaining for Myh3+ (grey) newly formed myofibers, Myh4+ (black) type-IIb fast twitch myofibers, and necrotic myofibers infiltrated with mouse immunoglobulin G (msIgG+, white) on frozen sections from regenerating TA muscles of mdx mice 10 days post cardiotoxin-induced injury and treated with DMSO (vehicle), or PTP1b inhibitor.
**Figure 13(C)** shows the relative mass of regenerating TA muscles of mdx mice 10 days post cardiotoxin-induced injury and treated with DMSO (vehicle), or PTP1b inhibitor normalized to their contralateral legs.
**Figure 13(D)** shows the relative average minimum Feret of myofibers from regenerating TA muscles of mdx mice 10 days post cardiotoxin-induced injury and treated with DMSO (vehicle), or PTP1b inhibitor.
**Figure 13(E)** shows the distribution of minimum Feret of myofibers from regenerating TA muscles of mdx mice 10 days post cardiotoxin-induced injury and treated with DMSO (vehicle), or PTP1b inhibitor compared to uninjured contralateral legs.
**Figure 13(F)** shows the quantification of Pax7+ cells on frozen sections from regenerating mdx TA muscles 10 days post cardiotoxin-induced injury and treated with DMSO (vehicle), or PTP1b inhibitor. The error bars for FIG. 13 represent means ± SEM; p-values: *=< 0.05; **=< 0.01; ***=< 0.005.
**Figure 14** shows a schematic of pharmacological inhibition of PTP1b to stimulate EGFR signaling in muscle stem cells.

### DETAILED DESCRIPTION

Generally, described herein is a method for stimulating asymmetric division of at least some satellite stem cells in a patient suffering from a disease or disorder characterized by satellite cells having an inability, or a reduced ability, to assemble a functional dystrophin-associated glycoprotein complex (DGC) that results in an inability, or reduced ability, to establish cell polarity. The disease or disorder may be muscular dystrophy. The method includes administering to the patient a sufficient amount of an epidermal growth factor receptor (EGFR) pathway activator to stimulate asymmetric division of at least some satellite stem cells.

In the present disclosure, a "disease or disorder characterized by satellite cells having an inability, or a reduced ability, to assemble a functional dystrophin-associated glycoprotein complex (DGC) that results in an inability, or reduced ability, to establish cell polarity" may, for brevity, be referred to as "the disease or disorder" or "a disease or disorder characterized by satellite cells having an inability, or a reduced ability, to establish cell polarity".

In some examples, the EGFR pathway activator is administered in an amount sufficient to increase levels of phosphorylated EGFR in the satellite stem cells by at least 20% in comparison to the levels of phosphorylated EGFR present before administration of the EGFR pathway activator. In some examples, the EGFR pathway activator is administered in an amount sufficient to activate Aurka kinase A and induce satellite cell apicobasal polarity. In some examples, the EGFR pathway activator is administered in an amount sufficient to increase the proportion of asymmetric divisions in the satellite stem cells to a level that is at least 50% of the proportion of asymmetric divisions in a population of normal satellite stem cells. In some specific examples, the level is at least 80% of the normal satellite stem cells. In some examples, the EGFR pathway activator is administered in an amount sufficient to increase the number of asymmetric divisions of the satellite stem cells per myofiber by at least 85% over the number of asymmetric divisions of the satellite stem cells per myofiber before administration of the EGFR pathway activator. In some specific examples, the increase was up to 150%. For example, if the average number of asymmetric divisions per myofiber before administration is 0.235 and the average number of asymmetric divisions per myofiber after administration of the EGFR pathway activator is 0.463, then the increase is 97%.

In the context of the present disclosure when the disease or disorder is muscular dystrophy, symmetric division of a satellite stem cell should be understood to refer to cell division that generates a daughter satellite stem cell characterized by a lack of expression of Myf5 protein and MyoD protein, and a daughter cell characterised by expression of Myf5 protein, MyoD protein, or both Myf5 protein and MyoD protein. The daughter cell characterised by expression of MYF5 protein and/or MyoD protein may be referred to as a committed myogenic progenitor cell.

The disease or disorder is characterized by satellite stem cells having an inability, or a reduced ability, to establish cell polarity. The ability of a satellite stem cell to establish cell polarity may be determined by measuring the angle between the basal lamina and the alignment between pairs of centrosomes. A method of measuring the angle is described in Dumont et al. "Dystrophin expression in muscle stem cells regulates their polarity and asymmetric division" Nat. Med. (2015) 21:1455-63. If the mitotic plane can be oriented in the apicobasal orientation (approximately 90 degrees to the basal lamina), the cell would be understood to have the ability to establish cell polarity and be able to undergo asymmetric division. Although a single satellite stem cell may either be able to, or be unable to, establish cell polarity, a population of satellite stem cells may have, as a whole, a reduced ability to establish cell polarity. For example, in a population of satellite stem cells, 50% may be able to establish cell polarity and 50% may not be able to establish cell polarity. In such a situation, the population could be referred to as having a 50% reduction in ability to establish cell polarity. This inability, or reduced ability, to establish cell polarity may be due to a loss of, or a reduced production of, dystrophin by the satellite stem cells. In the invention provided herein, the disease or disorder is muscular dystrophy, such as Duchenne muscular dystrophy, a dystroglycanopathy, a congenital muscular dystrophy, or a limb-girdle muscular dystrophy.

An EGFR pathway activator may be a direct activator of the EGFR pathway, or an indirect activator of the EGFR pathway. In the invention provided herein, the EGFR pathway activator is a direct activator of the EGFR pathway.

A direct activator would be understood to be a compound, peptide, or protein that acts by directly binding the EGF receptor on the cell surface of the satellite stem cell to activate the EGFR-Aurka signaling pathway. Examples of direct activators that may be used to activate the EGFR-Aurka pathway include: epidermal growth factor (EGF), TGF-alpha, amphiregulin, heparin-binding EGF-like growth factor (HB-EGF), betacellulin, epiregulin, any variants thereof having a sequence that is at least 80% identical to the reference sequence, and any fragments thereof that maintain at least some activating activity. Human EGF protein may have a sequence as set out in Uniprot entry number P01133. Human TGFA protein may have a sequence as set out in Uniprot entry number P01135. Human amphiregulin protein may have a sequence as set out in Uniprot entry number P15514. Human HG-EGF protein may have a sequence as set out in Uniprot entry number O99075. Human betacellulin protein may have a sequence as set out in Uniprot entry number P35070. Human epiregulin protein may have a sequence as set out in Uniprot entry number 014944.

The direct activator may alternatively be an anti-EGFR antibody, or an antigen-binding fragment of an anti-EGFR antibodies, capable of promoting receptor dimerization. The anti-EGFR antibody may bind to an EGF-binding domain on the EGFR. The antigen-binding fragment of the anti-EGFR antibody may be a cell-penetrating nanobody that binds an intracellular kinase domain of EGFR.

An indirect activator would be understood to be a compound, peptide, or protein that acts by interacting with a protein that is not the EGF receptor, but that is involved with a protein or pathway that interacts with the EGFR-Aurka signalling pathway. For example, protein tyrosine phosphatase 1b (PTP1b, also known as Ptpn1) dephosphorylates EGFR prior to recycling or degredation of the EGFR. Human PTP1b protein may have a sequence as set out in Uniprot entry number P18031. Inhibition of PTP1b activity reduces the overall rate of the dephosphorylation, which results in more activation of the EGFR pathway than is seen in the absence of the PTP1b inhibitor. An inhibitor of PTP1b dephosphorylation would be understood to be an example of an indirect activator of the EGFR pathway since it interacts with PTP1b and the PTP1b affects the relative amount of EGFR to activated EGFR.

The indirect EFGR pathway activator may be: a PTP inhibitor, preferably a PTP1b inhibitor, and even more preferably the PTP1b inhibitor has IC50's for SHP1 and SHP2 phosphatases that are more than 2-orders of magnitude greater than the IC50 for PTP1b. Such a PTP1b inhibitor is desirable since SHP1 and SHP2 phosphatases are involved in EGFR signaling.

The PTP1b inhibitor may be: 3-(3,5-dibromo-4-hydroxybenzoyl)-2-ethyl-N-[4-[(2-thiazolylamino)sulfonyl]phenyl]-6-benzofuransulfonamide; a 2-(oxalylamino)-4,5,6,7-tetrahydrothieno[2,3-c]pyridine-3-carboxylic acid salt; an N-alkyl-2-(oxalylamino)-4,5,6,7-tetrahydrothieno[2,3-c]pyridine-3-carboxylic acid salt; or a prodrug thereof. In some specific examples, the PTP1b inhibitor may be: 2-(oxalylamino)-4,5,6,7-tetrahydrothieno[2,3-c]pyridine-3-carboxylic acid hydrochloride ("TCS 401"); 6-methyl-2-(oxalylamino)-4,5,6,7-tetrahydrothieno[2, 3-c]pyridine-3-carboxylic acid trifluoroacetic acid salt ("BML-267"); or a prodrug thereof.

A "prodrug" would be understood to refer to compound that is metabolized, after administration, into the active drug compound. For example, a prodrug may be metabolized by chemical or enzymatic hydrolysis of a pro-drug ester to the respective carboxylic acid compound. Esters that may be metabolized after administration to a patient include alkyl esters (such as C₂-C₈ alkyl esters).

Satellite stem cells, or satellite cells, give rise to precursor cells capable of differentiating into skeletal muscle cells. Satellite stem cells can give rise to satellite cells and these both give rise to progenitors that fuse to form differentiated skeletal muscle cells. Symmetric division of a satellite cell gives rise to two satellite cells, while asymmetric division of a satellite cell gives rise to one satellite cell and one committed myogenic progenitor cell. Satellite stem cells in muscular dystrophy are characterized by expression of paired box 7 (PAX7) protein and EGFR protein, and by a lack of expression of myogenic factor 5 (MYF5) protein and MyoD protein. In the context of the present disclosure, expression of PAX7 and EGFR protein should be understood to refer to a level of protein expression that is substantially equal to the protein expression level in a satellite cell from a person without the disease or disorder. The PAX7 and/or EGFR protein may be detected using immunofluorescence.

The present disclosure provides an EGFR pathway activator formulated for administration to a patient suffering from a disease or disorder characterized by satellite cells having an inability, or a reduced ability, to establish cell polarity, in an amount sufficient to stimulate asymmetric division of at least some satellite stem cells in the patient. In the invention, the disease or disorder is muscular dystrophy.

Described herein is a use of an EGFR pathway activator for stimulating asymmetric division of at least some satellite stem cells in a patient suffering from a disease or disorder characterized by satellite cells having an inability, or a reduced ability, to establish cell polarity. The disease or disorder may be muscular dystrophy.

Also described is a use of an EGFR pathway activator in the manufacture of a medicament for stimulating asymmetric division of at least some satellite stem cells in a patient suffering from a disease or disorder characterized by satellite cells having an inability, or a reduced ability, to establish cell polarity. The disease or disorder may be muscular dystrophy.

In a further aspect, the present disclosure provides an EGFR pathway activator for use in a method of stimulating asymmetric division of at least some satellite stem cells in a patient suffering from a disease or disorder characterized by satellite cells having an inability, or a reduced ability, to establish cell polarity. In the invention, the disease or disorder is muscular dystrophy.

Stimulating asymmetric division of at least some satellite stem cells in a patient suffering from the disease or disorder may improve or rescue satellite cell function, improve or restore the proportion of asymmetric divisions of satellite stem cells, or any combination thereof. In the context of the present disclosure, the term "improve" should be understood to be in comparison to a level, function, or amount prior to administration of the EGFR pathway activator. In the context of the present disclosure, the terms "rescue" and "restore" should be understood to mean changing a level, function, or amount so that it is approaching or is nearly equal to the level, function, or amount in a person without the disease or disorder. Satellite stem cell function may be indirectly determined by measuring differentiation-competent progenitors, muscle regeneration, or force generation by muscles. Measuring differentiation-competent progenitors may be achieved by measuring the density of Pax7+ and MyoD+ cells, or of Pax7+ and Myg+ (Myogenin) cells, in a histological section as detected by immunofluorescence. Measuring myofiber damage may be achieved counting the number of myofibers exhibiting IgG infiltration. Measuring myofiber regeneration may be achieved by counting the number of myofibers exhibiting Myh3 expression, which marks newly regenerated myofibers. Methods of determining satellite stem cell function are discussed in von Maltzahn, J. et al. "Wnt7a treatment ameliorates muscular dystrophy." Proc Natl Acad Sci USA (2012) 109:20614-20619.

For example, restoring or rescuing satellite cell function in a cell from a patient with the disease or disorder may be understood to refer to permanently or temporarily altering at least some satellite stem cells that have an inability to establish cell polarity such that the restored or rescued satellite stem cells have an ability to establish cell polarity that approaches, or is nearly equal to, the corresponding ability of satellite stem cells from a person without the disease or disorder.

Stimulating asymmetric division of at least some satellite stem cells in the patient may reduce the severity of one or more symptoms of the disease or disorder in the patient. For example, a patient with muscular dystrophy may have a reduced disease progression, a reduced loss rate of muscle strength, an increase in muscle strength, or a combination thereof. For example, a patient with muscular dystrophy may have an improved ability to breath, swallow, grip, ambulate, or a combination thereof.

EGFR pathway activators may be identified in a method of screening potential activators. The method includes visualizing isolated, cultured, and stained Flexor Digitorum Brevis (FDB) myofibers from Myf5-Cre/ROSA26-YFP mice in a plurality of Z-stack planes to create a projection image. Muscle satellite cells are identified in the projection image based on Pax7 expression. Of the identified muscle satellite cells: (i) satellite stem cells are identified based on the absence of YFP expression, and (ii) committed satellite progenitor cells are identified based on yellow fluorescence protein (YFP) expression. A potential EGFR pathway activator is administered to the FDB myofibers. The number of symmetric divisions of satellite myogenic cells, identified by the production of two YFP(+) satellite cells, are counted; the number of symmetric satellite stem cell divisions, identified by the production of two YFP(-) satellite cells, are counted; and the number of asymmetric satellite cell divisions, identified by the production of one YFP(+) and one YFP(-) satellite cell, are counted. The potential EGFR pathway activator is identified as an EGFR pathway activator when the percent of YFP(-) divisions that are asymmetric divisions is greater than the corresponding percent in a control sample. The expression "YFP(-) divisions" refers to all cell divisions where one of the daughter cells is YFP(-).

The method may be automated and adapted for high throughput screening using a ThermoFisher Scientific^{™} Cellomics platform, which combines fluorescence microscopy, image processing, automated cellular measurements, and informatics tools for high content analysis. Using a Cellomics platform, detection of satellite stem cells may be achieved with at least a 90% detection rate, a false positive detection rate of less than about 2%, and a z-factor score of 0.93. Detection of discrete stem cell divisions may be achieved with at least a 78% detection rate, a false positive detection rate of less than about 2% and a z-factor score of 0.81.

### Examples

The inventors determined that EGFR signaling regulates asymmetric satellite stem cell divisions, and EGF treatment stimulates asymmetric division in dystrophin-deficient satellite stem cells. The authors of the present disclosure also determined that EGF treatment resulted in an increase in the number of myogenic progenitor cells *in vivo.* The inventors further determined that inhibition of PTP, especially PTP1b, may reduce EGFR recycling or degradation, increase EGFR activity, and thereby stimulate asymmetric division in dystrophin-deficient satellite stem cells. The inventors also determined that PTP1b inhibition resulted in an increase in the number of myogenic progenitor cells *in vivo.* These results are discussed in greater detail below.

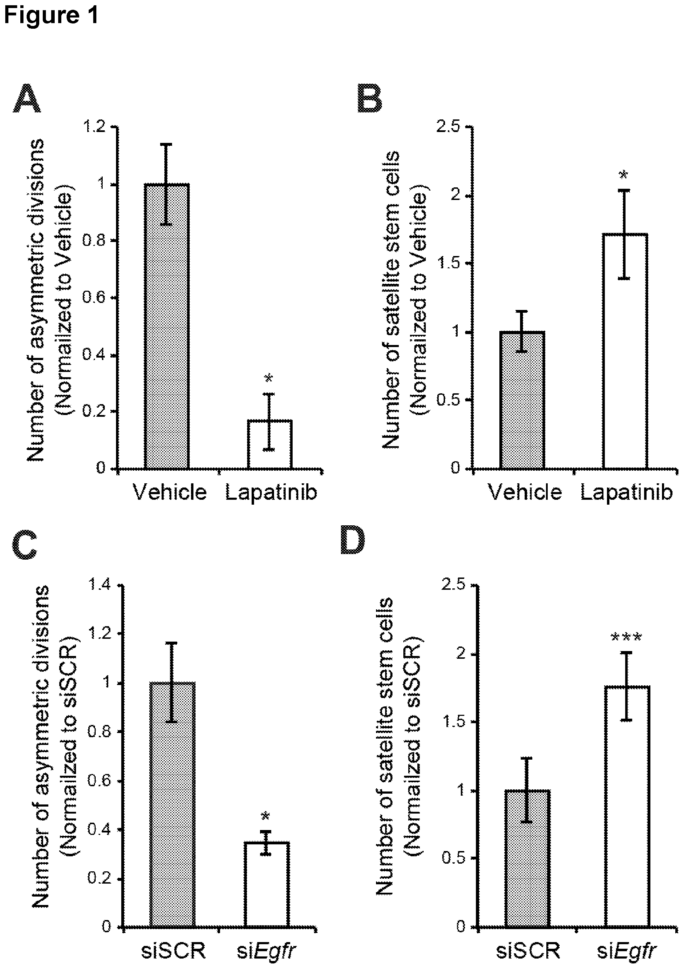

**EGFR Signaling Regulates Asymmetric Satellite Stem Cell Divisions.** To assess the role of EGFR in asymmetric division, the inventors cultured satellite cells in myofibers isolated from Extensor digitorum longus (EDL) muscles from Myf5-Cre/R26R-eYFP mice and performed both loss-of-function and gain-of-function experiments. Isolated myofibers were first cultured for 42h with and without treatment with the Lapatinib, a specific inhibitor of EGFR signaling. The concentration of Lapatinib used in the screen completely abolishes EGFR signaling (data not shown). Inhibition of EGFR signaling resulted in marked shift towards satellite stem cell symmetric divisions as evidenced by an 83% decrease in the rate of asymmetric divisions (Figure 1A). Inhibition of EGFR signaling thus resulted in a 71% increase in the number of satellite stem cells (Figure 1B).

To validate that EGFR is indeed the specific gene target of Lapatinib, siRNA against EGFR (siEGFR) was transfected in satellite cells on single EDL myofibers isolated from Myf5-Cre/R26R-eYFP mice. Similar to pharmacological inhibition, siEGFR reduced the rate of asymmetric division by 65% compared to transfection with a scrambled siRNA (siSCR) (Figure 1C). Transfection with siEGFR also increased the rate of symmetric divisions and led to a 76% increase in satellite stem cell numbers (Figure 1D). Therefore, the inventors conclude that EGFR plays a direct role in controlling the rate of asymmetric division in satellite stem cells.

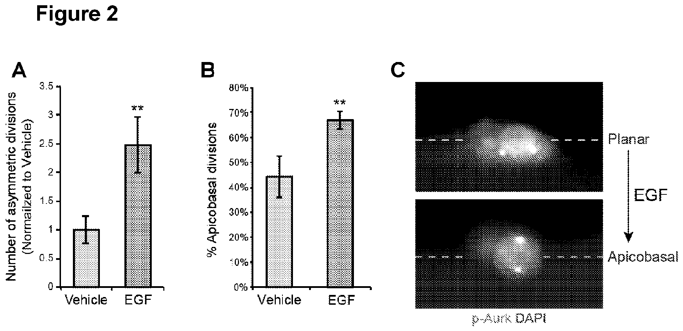

To investigate whether activation of EGFR signaling acts as a driver of asymmetric division, recombinant EGF protein was supplemented to the culture media of single EDL myofibers isolated from Myf5-Cre/R26R-eYFP mice (Figure 2). Notably, the inventors observed that EGF treatment led to a 2.5-fold increase in the rate of asymmetric satellite stem cell division (Figure 2A). Recent studies of polarized epithelial MDCK cells have identified a role for EGFR in orienting the mitotic axis along the apicobasal axis. Moreover, asymmetric satellite cell divisions occur in an apicobasal orientation. The inventors cultured single EDL myofibers for 36 h and satellite cell centrosomes were detected by immunostaining for phosphorylated aurora kinase (p-Aurk). Around 5% of cells were labeled with p-Aurk at 36 h irrespective of EGF treatment. EGF treatment resulted in a 50% increase in the proportion of mitotic cells aligned along the apicobasal axis (Figure 2B and 2C). Without wishing to be bound by theory, it is believed that EGF stimulation of satellite cells stimulates a change in mitotic orientation by the recruitment of centrosomes along the polarized p-EGFR signal.

**EGF Treatment Rescues the Polarity Deficits in Dystrophin-Deficient Satellite Cells.** Loss of dystrophin in Duchenne muscular dystrophy (DMD) causes a polarity deficit in satellite cells in *mdx* mice, a mouse model of DMD. Satellite stem cells lacking dystrophin exhibit a 10-fold reduction in the number of asymmetric divisions, resulting in diminished generation of Myog-expressing myogenic progenitors and delayed regeneration.

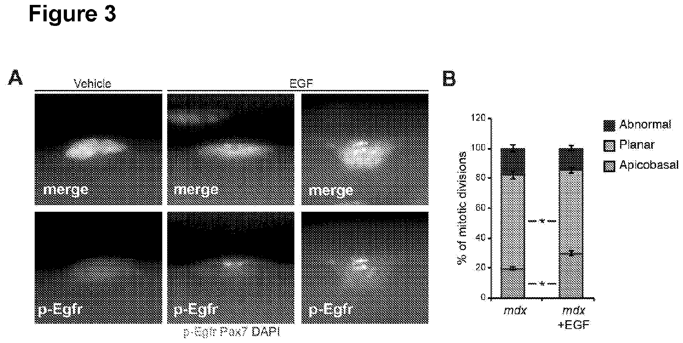

To establish whether EGFR localization and activation are affected by the loss of dystrophin, single EDL myofibers from *mdx* mice were immunostained for phosphorylated EGFR (p-EGFR) after 1h with or without recombinant EGF stimulation. P-EGFR was only observed in *mdx* fibers treated with EGF (Figure 3A). Moreover, comparable to wild type (WT) cells, p-EGFR was similarly localized in 'streak'-like structures on the basal surface of *mdx* cells. Therefore, the inventors conclude that that EGFR signaling occurs normally in *mdx* satellite cells.

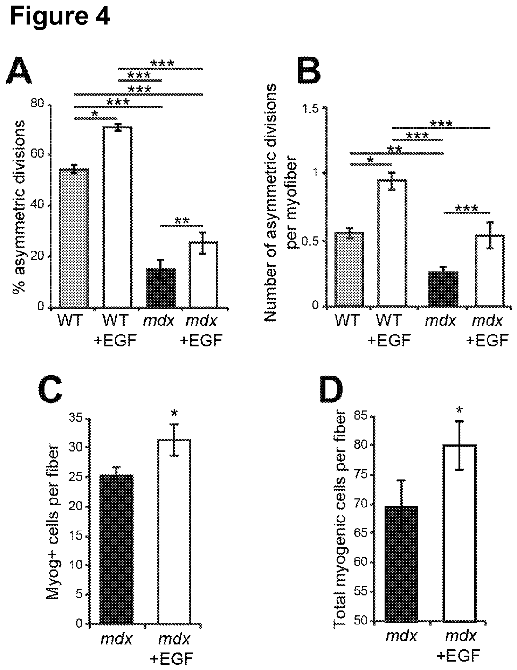

To assess whether EGFR signaling would stimulate the asymmetric division of dystrophin-deficient satellite stem cells, EDL myofibers were isolated from WT and *mdx* Myf5-Cre/R26R-eYFP mice and cultured for 42h with and without recombinant EGF stimulation. The rate of asymmetric *mdx* satellite stem cell divisions is significantly reduced relative to WT satellite cells. EGF treatment of WT satellite cells resulted in a 29% increase in asymmetric division. EGF stimulation of *mdx* satellite cells resulted in a 67% increase in the rate of asymmetric division. EGF stimulation of EGFR signaling was able to simulate asymmetric division of dystrophin-deficient satellite cells (Figure 3B and 4A).

Similar to WT satellite cells, EGF treatment of *mdx* satellite cells induced increased numbers of apicobasal orientated mitotic centrosomes. However, the rate of abnormal cell divisions in *mdx* satellite cells as evidenced by abnormal patterns of p-Aurk staining was unaffected by EGF stimulation. This observation implies that cell cycle dysregulation is not completely rescued by polarity signaling alone. However, the increased rate of asymmetric divisions suggested that EGF-treatment could ameliorate the reduced generation observed in regenerating *mdx* muscle. Therefore, to assess whether EGF-driven asymmetric divisions could ameliorate the reduced generation of myogenic progenitors in *mdx* cultures, single EDL myofibers from *mdx* mice were cultured for 72h and immunostained for the expression of Myog. Notably, EGF stimulation of *mdx* satellite cells led to a 2-fold increase in the absolute number of asymmetric satellite stem cell divisions after 42h of myofiber culture (Figure 4B), and a 20% increase in the number of MyoG-expressing cells after 72h of myofiber culture (Figure 4C). EGF treatment increased the total numbers of myogenic cells by about 14% (Figure 4D).

**EGF Treatment Enhances Regeneration of *mdx* Muscle.** The inventors found that EGF treatment stimulated an increase in dystrophin-deficient satellite stem cell asymmetric division, and increased numbers of progenitors on cultured myofibers.

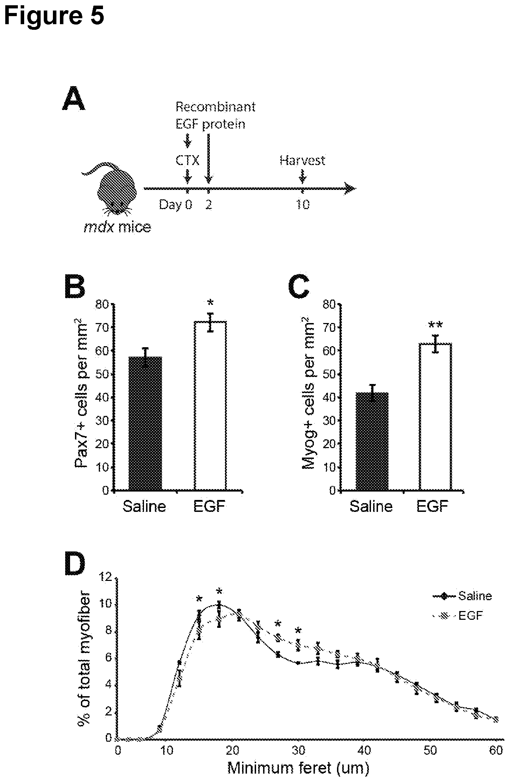

Previously, the inventors observed that dystrophin- or Dag1-deficient muscles generate reduced numbers of Pax7-expressing and Myog-expressing cells following CTX-injury. To assess the effect of EGF treatment on dystrophin-deficient satellite cells in vivo, 10 ng of recombinant EGF protein was intramuscularly (IM) injected at the time of CTX-induced injury and 2d after the injury (Figure 5A). It was observed that EGF-injection into injured *mdx* muscles resulted in a 26% increase in Pax7-expressing cells (Figure 5B), and a 50% increase in the numbers of Myog-expressing cells (Figure 5C). Moreover, regenerating myofibers in EGF-treated muscles exhibited an increase in Feret's diameter compared to vehicle-injected controls (Figure fD). These results suggest that intramuscular supplementation with recombinant EGF increases the productive generation of myogenic progenitors and thus enhances regeneration of *mdx* muscle.

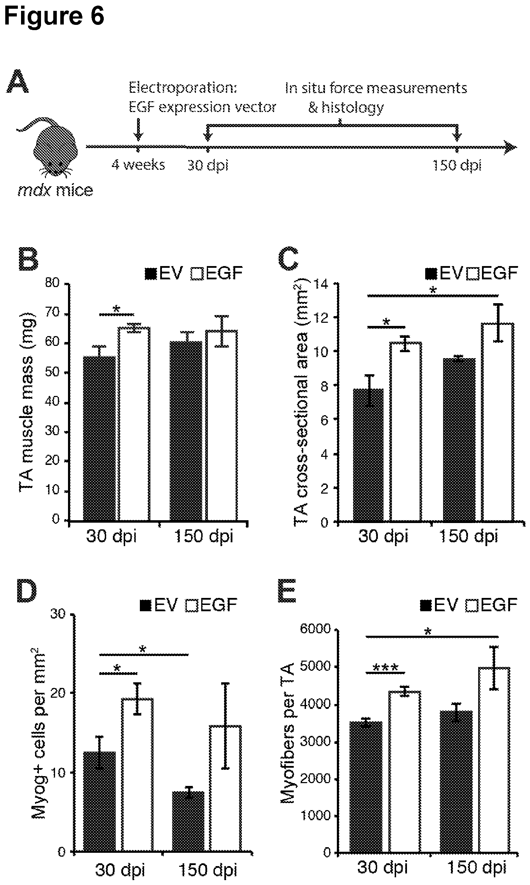

To address the effect of long-term EGF treatment, the inventors electroporated an expression plasmid containing the human EGF cDNA (Sun et al., 2015) into *mdx* tibialis anterior (TA) muscles. (Figure 6A). Strikingly, *mdx* muscles electroporated with the EGF expression vector exhibited an 18% increase in mass by 30 d.p.i. (Figure 6B). This increase in muscle mass is reflected in the overall cross-sectional area of the EGF-electroporated TA muscles compared to the empty-vector controls (Figure 6C).

Similar to effects we observed with short-term EGF treatment, electroporation with the EGF vector boosted the number of Myog+ myogenic progenitors at 30 d.p.i. and maintained their numbers even 150 d.p.i. whereas fewer Myog+ cells were found in the empty vector controls (Figure 6D). Surprisingly, the difference in Myog+ cells did not contribute to increased hypertrophy. Instead, we find a significant increase in the number of myofibers in the muscles electroporated with the EGF vector consistently at 30 and 150 d.p.i. (Figure 6E. 7A, 7B).

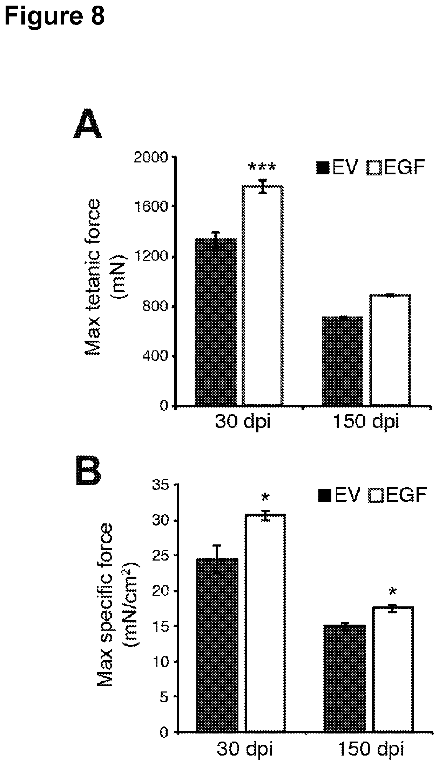

To measure the impact of these histological changes at the level of muscle function, we performed *in situ* measurements of TA muscle force generation. Strikingly, TA muscles electroporated with the EGF expression vector generated 32% greater force at tetanus compared to those electroporated with the empty vector at 30 d.p.i. (Figure 8A). Moreover, after normalizing to the physiological cross-sectional area of the muscle, we found that muscles electroporated with the EGF vector had 25% higher specific force at 30 d.p.i., which remained at 17% higher compared to the empty vector muscles at 150 d.p.i. (Figure 8B).

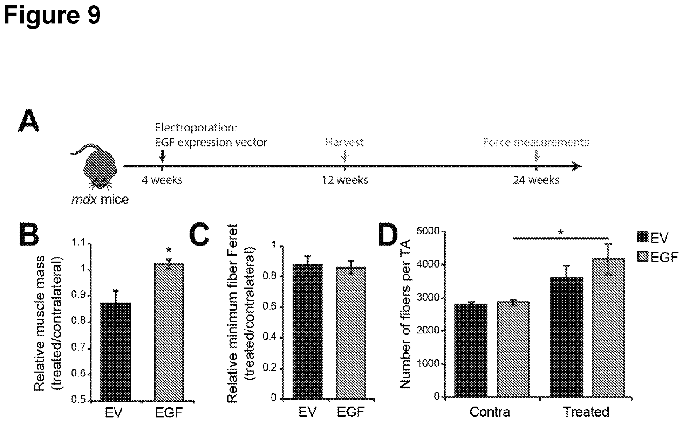

To address the effect of short-term EGF treatment, the inventors electroporated an expression plasmid containing the human EGF cDNA (Sun et al., 2015) into *mdx* tibialis anterior (TA) muscles. TA muscles were electroporated at 4 weeks of age, during the onset of muscle degeneration, and collected 24 weeks later (Figure 9A). Strikingly, *mdx* muscles electroporated with the EGF expression vector exhibited a 17% increase in mass (Figure 9B). While no difference in Feret's diameter of myofibers was observed (Figure 9C), the inventors found a 22% increase in the numbers of myofibers (Figure 9D).

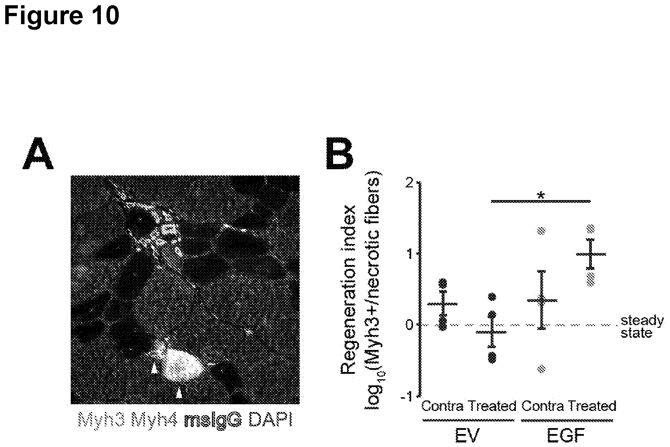

Since *mdx* muscles reside in a balance between degeneration and regeneration, muscle sections from the electroporated mice were quantified for newly formed embryonic Myosin Heavy Chain (Myh3)-expressing and necrotic myofibers infiltrated with serum IgG (mslgG) (Figure 10A). The ratio between newly formed and necrotic myofibers represents a relative regeneration index that quantifies progression of the dystrophic phenotype (Figure 10B. Whereas contralateral TAs of these *mdx* mice maintained a steady state with regeneration index near 0.3 (Figure 10B, TAs electroporated with the empty vector on average had more necrotic myofibers than newly formed myofibers resulting in a mean regenerative index of -0.09 (Figure 10B. All the TAs electroporated with the EGF expression vector contained more Myh3+ myofibers than necrotic ones and exhibited a mean regeneration index of 0.99 (Figure 10B). The increased regenerative index in EGF electroporated muscles corresponded to increased number of myofibers per TA muscle. Consistent with a reduced dystrophic pathology, EGF electroporation reduced the progressive increase in fibrosis by 26% as measured by wheat germ agglutinin (WGA) staining (Figure 10A, 10B).

**Inhibiting PTP1b to Increase EGFR Activity.** Feedback mechanisms may suppress EGFR signaling activity. For example, phosphorylated EGFR is dephosphorylated prior to being recycled or degraded. This dephosphorylation event is carried out by protein tyrosine phosphatase 1b (PTP1b, also known as Ptpn1) and may be targeted by pharmacological inhibitors.

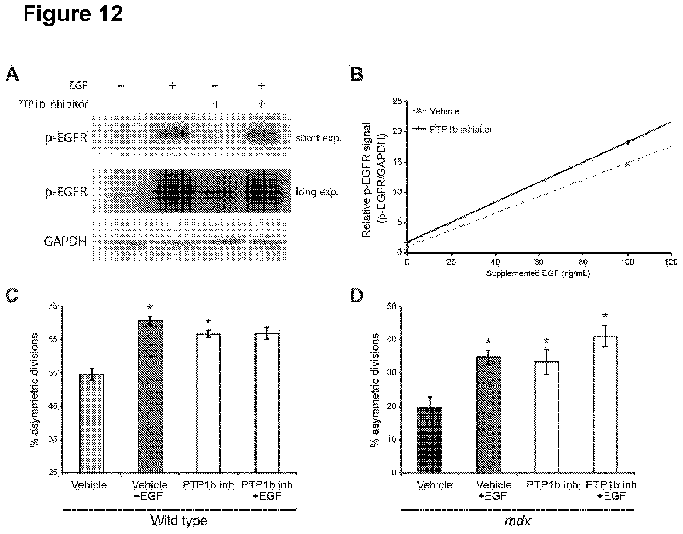

To assess whether PTP1b inhibition can further increase EGFR activity, the inventors of the present invention measured EGFR phosphorylation in serum-starved myoblasts re-fed with growth media, growth media supplemented with 3-(3,5-dibromo-4-hydroxy-benzoyl)-2-ethyl-benzofuran-6-sulfonicacid-(4-(thiazol-2-ylsulfamyl)-phenyl)-amide (a PTP1b inhibitor, CAS 765317-72-4), recombinant EGF, or both (Figure 12A). Quantification of the grey values of p-EGFR in these cells showed that PTP1b inhibition increased EGFR phosphorylation by 80% in growth media alone and 20% in the presence of added recombinant EGF (Figure 12A and 12B).

Since PTP1b inhibition is able to increase EGFR signaling in myoblasts, the inventors set out to address whether PTP1b inhibition affects satellite stem cell asymmetric division. EDL myofibers were isolated from wild type and *mdx* Myf5-Cre/R26R-eYFP mice and cultured for 42h in the presence of PTP1b inhibitor, recombinant EGF, or both (Figure 12C and 12D). Again, EGF alone was able to stimulate asymmetric divisions in both wild type and *mdx* satellite stem cells. PTP1b inhibition alone was sufficient to stimulate asymmetric division. The rates of asymmetric division were similar in wild type cells treated with EGF or PTP1b inhibitor. Table 1 depicts the quantification of asymmetric divisions relative to total satellite stem cell divisions in WT and mdx myofibers at 42h of culture in the presence of vehicle control, recombinant EGF, PTP1b inhibitor (CAS 765317-72-4), or both recombinant EGF and PTP1b inhibitor. S.E.M. is Standard Error of the Mean.

**TABLE 1**

| | Average number of asymmetric divisons/fiber | S.e.m. of number of asymmetric division/fiber | Proportion of YFP- divisions that are asymmetric divisions | S.e.m. of the proportion of asymmetric divisions |
|---|---|---|---|---|
| Wild type (WT) | 0.547 | 0.033 | 54.59% | 1.65% |
| WT + EGF | 0.939 | 0.064 | 70.86% | 1.28% |
| WT + PTP1b inhibitor | 0.988 | 0.150 | 66.70% | 0.99% |
| WT + EGF + PTP1b inhibitor | 0.945 | 0.100 | 66.77% | 1.84% |
| Mdx | 0.235 | 0.110 | 19.46% | 3.45% |
| mdx + EGF | 0.436 | 0.101 | 34.60% | 2.20% |
| mdx + PTP1b inhibitor | 0.463 | 0.111 | 33.33% | 3.85% |
| mdx + EGF + PTP1b inhibitor | 0.547 | 0.139 | 41.10% | 3.16% |

Without wishing to be bound by theory, these results suggest that PTP1b inhibition leads to an increase in active EGFR pathway, and induces muscle stem cell asymmetric division. The stimulation of asymmetric division in the satellite stem cells increase the number of committed myogenic progenitors, and accelerates muscle tissue regeneration.

**PTP1b Inhibition Enhances Regeneration of *mdx* Muscle.** Since PTP1b inhibition increased EGFR signaling and stimulated the asymmetric division of *mdx* satellite stem cells, the inventors set out to see if PTP1b inhibition may be used to stimulate asymmetric division *in vivo.*

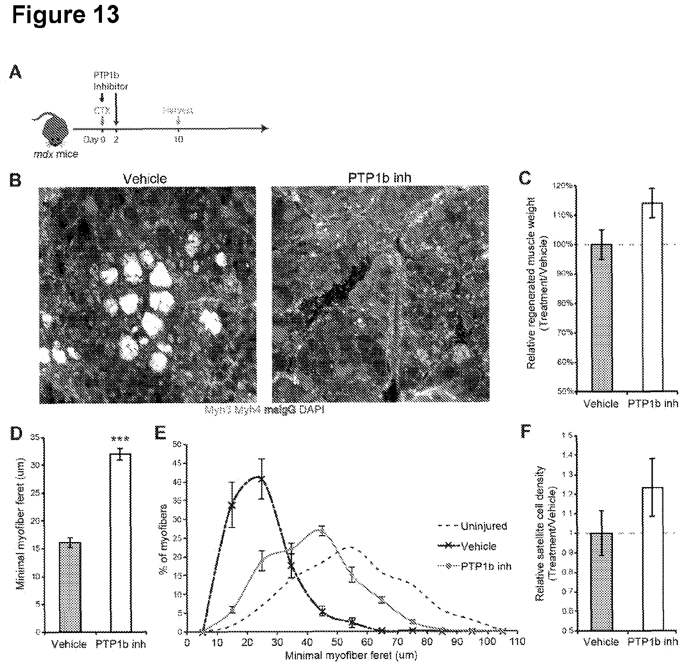

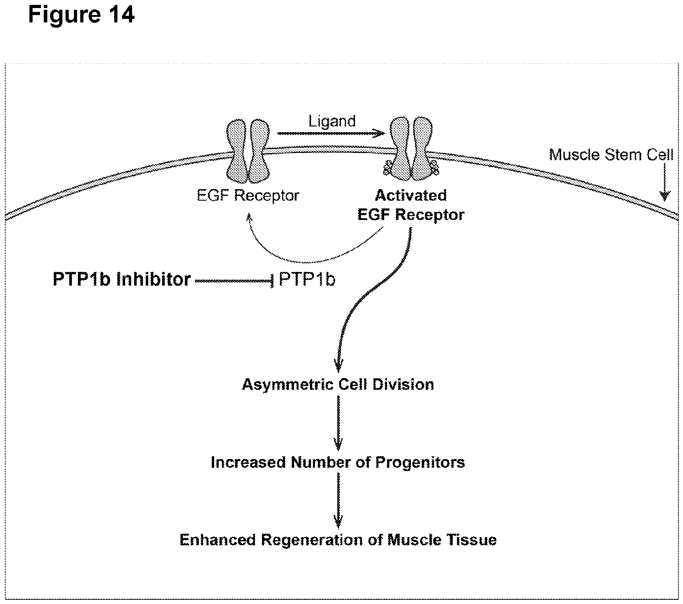

PTP1b inhibitor was concurrently administered with cardiotoxin into *mdx* TA muscles and supplemented again 2 days after the injury (Figure 13A). To assess the muscle stem cell function after injury and the regenerative capacity of the treated *mdx* muscles, the inventors examined the histology of the regenerating muscles 10 days post cardiotoxin injection. Whereas the control vehicle treated muscles still contained large areas of necrotic fibers (mslgG+), the muscles treated with PTP1b inhibitors were better regenerated as evidenced by the presence of 50% fewer Myh3-expressing myofibers (Figure 13B). The PTP1b inhibitor treated muscles also had a 14% increase in mass compared to vehicle treated controls (Figure 13C). Similar to treatment with recombinant EGF, the PTP1b inhibitor treated muscles contained myofibers with a 2-fold increase on average in Feret's diameters compared to the control muscles (Figure 13D and E). Finally, PTP1b inhibitor treated muscle contained increased numbers of satellite cells (Figure 13F). These findings are consistent with PTP1b inhibition inducing an increase in EGFR signaling and accelerated *de novo* myofiber formation in regenerating *mdx* muscles (Figure 14).

### Experimental Procedures

**Mice and animal care.** The following mouse lines were used: mdx, Myf5-Cre, and ROSA26-eYFP. All experiments were performed in accordance with University of Ottawa guidelines for animal handling and care. If not stated differently, 6-8 week old mice were used for all experiments.

**EDL fiber Culture and siRNA Transfection.** Myofiber culture was performed as described in Dumont, N.A., et al. "Dystrophin expression in muscle stem cells regulates their polarity and asymmetric division." Nat. Med. (2015) 21:1455-63. Briefly, EDL were carefully dissected and incubated in DMEM with 2% L-glutamine, 4.5% glucose, and 110 mg/mL sodium pyruvate (Gibco) containing 0.2% collagenase I (Sigma) for 45 min. Myofibers were isolated using gentle trituration with a glass pipet. Myofibers were cultured for 36, 42, or 72h in DMEM containing 20% FBS (Wisent), 1% chick embryo extract (MP Biomedicals), and 2.5 ng/ml bFGF (Cedarlane). For pharmacological inhibition, Lapatinib ditosylate (1 mM in DMSO; Santa Cruz Biotechnology), PTP1b inhibitor CAS 765317-72-4 (10 mM in DMSO; Santa Cruz Biotechnologies) was added to the culture medium for a final concentration of 1µM or 10µM, respectively; equal dilution of DMSO was used as vehicle control. For EGF treatment, human recombinant EGF (100 ng/µl in PBS with 0.1% BSA, Life Technologies) was added to the culture medium at 100 ng/mL.

To assess the signaling status of EGFR in quiescent satellite cells, EDL muscles were fixed in 4% PFA immediately post-dissection. Myofiber bundles were teased from the fixed muscles by tweezers. For studies pertaining to the activation of EGFR, myofibers were isolated in serum-free conditions with DMEM with 2% L-glutamine, 4.5% glucose, and 110 mg/mL sodium pyruvate (Gibco) and then treated with human recombinant EGF (100 ng/µl in PBS with 0.1% BSA Life Technologies) at 100 ng/mL for 1h, equal dilution of 0.1% BSA in PBS was used as vehicle control.

Transfection of satellite cells on myofibers was performed using lipofectamine RNAimax (Life Technologies) and validated Smartpool siRNAs for EGFR, Aurka, or scramble (SCR) (Dharmacon). Two transfections were performed at 4h and 16h after isolation of the myofibers as described in Le Grand, et al. (2009) Wnt7a activates the planar cell polarity pathway to drive the symmetric expansion of satellite stem cells. Cell Stem Cell 4, pp 535-547. Knockdown efficiencies of the siRNA were validated in myoblasts by western blot and qRT-PCR.

**Characterization of Satellite Cell Divisions.** The orientation of mitotic spindles in satellite cells was measured as described in Dumont et al. (2015). Single myofibers were fixed after 36 h of in vitro culture, as described above. Satellite cells were identified by Pax7 expression in the nucleus, as detected by immunofluorescene. Pax7 staining becomes cytoplasmic in mitotic satellite cells after the dissociation of the nuclear envelope, but is still discernable. Mitotic satellite cells were identified by positive p-Aurk staining, which labels cells from pro-metaphase to cytokinesis. Mitotic satellite cells with p-Aurk staining patterns that were not observed in WT cells, including monopolar, multipolar (>2), and abscission defects were quantified as abnormal. Mitotic orientations were manually counted according to the angle between the mitotic spindle and the tangential plane of the satellite cell's attachment point to the myofiber.

**Cell Culture and Co-Immunoprecipitation.** Satellite cells were isolated from WT mice by fluorescence activated cell sorting and cultured as primary myoblasts on collagen-coated plates in primary myoblast growth media (Ham's F10 media (Gibco) with 20% FBS (Wisent), 1% penicillin-streptomycin (Gibco) and 5 ng/mL bFGF (Cedarlane)). For EGF stimulation, myoblasts were serum starved for 1 h in Ham's F10 media and then refed in primary growth media with or without recombinant human EGF (100 ng/mL) for an additional hour.

**Immunoblotting.** Proteins were separated on 10% SDS-PAGE and transferred to Immobilon-P PVDF membrane (EMD Millipore). Membranes were probed with primary antibodies, followed by light chain specific HRP-conjugated secondary antibodies at 1:5000 (Bio-Rad) and developed using Immobilon Western HRP substrate (EMD Millipore). Membranes were visualized using FluorChem HD2 (Alpha Innotech) or exposed to BIOMAX film (Eastman Kodak).

**Electroporation and Cardiotoxin Injury.** Intramuscular (i.m.) cardiotoxin injections (Latoxan, 50 µl of 10µM solution in saline) were injected directly into the right TA muscle through the skin under general anesthesia. For pharmacological inhibition, Lapatinib ditosylate (1 mM in DMSO; Santa Cruz Biotechnology), the PTP1b inhibitor CAS 765317-72-4 (10 mM in DMSO; Santa Cruz Biotechnologies) was mixed into the cardiotoxin solution for a final concentration of 1 µM or 20 µM, respectively; equal dilution of DMSO was used as vehicle control. Supplemental injection of inhibitors (20 µL of 2.5 µM of Lapatibin or 50 µM of PTP1b inhibitor diluted in saline) was performed 2 days after cardiotoxin injection. For recombinant EGF injections, 10 ng of human recombinant EGF (100 ng/µL in saline, Life Technologies) was mixed into 50 µL of cardiotoxin solution or 20 µL of saline, equal volumes of saline was used as vehicle control.

Electroporations were performed as described in Bentzinger, C.F., et al. "Fibronectin regulates Wnt7a signaling and satellite cell expansion." Cell Stem Cell. (2013) 12:75-87. 30 µg of purified endotoxin-free expression plasmid in saline was injected into the right hind TA muscle of 4 week old mdx mice through the skin under general anesthesia. Immediately after injection, electric stimulation was applied to the TA by a pulse generator (ECM 830, BTX) of 100-150 volts for 6 pulses, with a fixed duration of 20 ms and an interval of 200 ms using 5 mm needle electrodes (BTX).

**Immunostaining and Antibodies.** EDL myofibers were fixed for 10 min in 2% PFA and washed with PBS. Fibers were blocked and permeablized in horse serum blocking buffer (5% horse serum, 1% BSA (Sigma), and 0.5% Triton X-100 (Sigma) in PBS) for 1 h at room temperature or at 4 °C over night. Primary antibodies were applied in blocking solution for 2h at room temperature or at 4°C over night. Samples were subsequently washed with PBS and stained with appropriate fluorescently labeled secondary antibodies (Alexa fluor 488, 546, or 647) for 1 h at room temperature. After washing with PBS, samples were mounted with Permafluor (Fisher).

Muscle samples were embedded in OCT embedding compound and frozen in liquid nitrogen cooled isopentane and cryosectioned in 12-µm slices. Cross-sections were washed once with PBS and fixed in 2% PFA 10min, permeabilized with 0.1% Triton X-100 / 0.15 M Glycine / PBS for 10 min and extensively washed with PBS. Samples were blocked using M.O.M. Blocking reagent (Vector) for 2 h followed by additional blocking in 5% NGS/2%BSA at 4°C over night. Primary antibodies were applied in blocking at 4 °C over night. Samples were washed extensively in PBS and secondary antibodies were applied in PBS (Alexa Flour 546, 647) for 1 h at room temperature. Following PBS washes, cross-sections were counterstained with DAPI (4',6-diamidino-2-phenylindole) for 10 min and mounted with Permaflour (Fisher).

Antibodies were as follows: mouse anti-Pax7 (DSHB), chicken anti-GFP (cat# ab13970, Abcam), rabbit anti-EGFR (cat# 4267S, Cell Signaling technology), rabbit anti-phospho-EGFR Y1068 (cat# 3777S, Cell Signaling technology), rabbit anti-phospho-Aurk (cat# 2914S, Cell Signaling technology), and rabbit anti-myogenin (M225, cat# sc-576, Santa Cruz).

**Histological Analysis of Muscle Sections.** For Fiber type analysis, non-fixed samples were washed with PBS and blocked in 10% normal goat serum (NGS) for 1 hr at room temperature. Primary antibodies were applied in 10% NGS for 2 h at room temperature. Sections were washed extensively with PBS and secondary antibodies were applied in PBS for 1 h at room temperature. Following PBS washes, sections were counterstained with DAPI for 10 min and mounted with Permaflour. Antibodies were as follows: mouse anti-MyH3 (clone F1.652, DSHB), mouse anti-MyH4 (clone BF-F3, DSHB).

Regeneration index of mdx muscles were calculated as "regenerative index = log10 (number of newly formed Myh3 myofibers / number of necrotic IgG myofibers)". Log transformation was used to linearize the scale of the index. Statistical tests were performed on the geometric mean of the index.

For analysis of myofiber Feret's diameter, non-fixed samples were washed with PBS and stained with Wheat Germ Agglutinin Alexa 647 conjugate (Fisher) for 1 h at room temperature. Samples were washed once with PBS and counterstained with DAPI. Samples were washed with PBS and mounted with Permaflour. Images were taken immediately following staining. Minimum fiber Feret measurement was performed using the SMASH software in MATLAB 2015a as described in Smith, L.R., and Barton, E.R. (2014) *SMASH - semi-automatic muscle analysis using segmentation of histology: a MATLAB application.* Skelet. Muscle 4, 21. Samples with significant staining artifacts were excluded from automated analyses.

**Statistical Analysis.** Compiled data were expressed as mean ± standard error of the mean (SEM). Experiments were performed with a minimum of three biological replicates. For statistical comparisons of two conditions, the Student's t-test was used. Paired tests were used for biologically matched samples. Unpaired tests were used to compare unrelated samples. The level of significance is indicated as follows: * p < 0.05, ** p < 0.01, *** p < 0.005.

**Identification of EGFR pathway activators.** Flexor Digitorum brevis (FDB) fibers were isolated from Myf5-Cre/Rosa-YFP mice and digested in 0.25% Collagenase Type I (Sigma) in DMEM (Dulbecco's modified Eagle's medium; high glucose, L-glutamine with 110 mg/ml sodium pyruvate) for 2.5-3 h. Myofibers were dissociated using gentle trituration with a glass pipette.

Myofibers are cultured at 37°C, 5% CO₂ for 42 h in myofiber culture media (DMEM supplemented with 20% FBS(Wisent), 1% Chicken Embryo extract (MP Biomediacals), 1% Penicillin/Streptomycin (Cederlane) and 2.5 ng/ml basic Fibroblast Growth Factor (Cedarlane).

Myofibers are first fixed in 2% Paraformaldehyde (PFA) for 5 mins. Myofibers are blocked for 1-2 h in 1% Bovine Serum Albumin, 0.5% TritonX-100, 5% HS and 0.01% Sodium Azide. Primary antibodies are applied in blocking solution for 2 h at room temperature or at 4 °C overnight. Myofibers are washed with PBS and stained with appropriate fluorescently labeled secondary antibodies (Alexa Fluor 488, 546) for 1 h at room temperature. Following washing with PBS, fiber nuclei are stained with DAPI for 5 min. The following antibodies are used: mouse anti-Pax7 (Developmental Studies Hybridoma Bank (DSHB)), chicken anti-GFP (cat. no. ab13970, Abcam).

Satellite cells are focused based on Channel 3 (DAPI) staining. Autofocus is performed by sampling seven (7) z-stacks taken at 13 µm intervals for an extended depth of field of 85.3 µm.

Z-stack images were consolidated to create a projection image with all satellite cells in focus. Image acquisition of a projection image for analysis was created from seven (7) Z-stack images taken at 6.5 µm intervals, for an extended depth of field of 45.5 µm at 200 (Objective 20x) magnification. The projection direction was centered, where the median stacks fall within the center of each myofiber. A maximum-minimum difference projection method that uses differences in pixel brightness between adjacent sources was used to reduce background. Further background subtraction was applied to each channel: low pass filter was applied to Channel 1 (Pax7) and Channel 3 (DAPI), while 3D surface fitting was applied to Channel 2 (YFP).

Potential satellite cells were first identified based on Channel 1 (Pax7) signal, and separated from other satellite cells based on intensity of fluorescence between cells. A threshold was applied for cells that are too dim to be considered satellite cells, and to prevent false positive selection of debris. Satellite cells were then validated based on area, shape, total intensity, average intensity, and variation of intensity. Finally, satellite cells were validated by Channel 3 (DAPI), which identified real nuclei based on total and average intensity of signal.

Data related to Well number, Field number, Cell number, cell coordinates (pixel count and X, Y coordinates), Average channel 1 intensity (Pax7), Average channel 2 intensity (YFP), and Average channel 3 Intensity (DAPI) was obtained and used to identify satellite cell couplets, and commitment status for each satellite cell.

This method was validated using EGF ligand as a known activator of asymmetric satellite cell divisions, using a PTP1b inhibitor as a known activator of asymmetric satellite cell divisions; and using Wnt7a ligand as a known activator of symmetric satellite cell divisions. The percentage of YFP(-) divisions that were asymmetric divisions was 71% with EGF ligand treated cells and 67% with the PTP1b treated cells vs. 55% with control treated cells. The percentage of YFP(-) divisions that were symmetric divisions was 78% with Wnt7a ligand treated cells vs. 62% with control treated cells. The percentage of YFP(-) divisions that were symmetric divisions was 29% with EGF ligand treated cells and 33% with the PTP1b treated cells vs. 45% with control treated cells.

In the preceding description, Uniprot entry numbers have been used to further define proteins that are identified by their commonly used names. The amino acid sequences, and the natural variants, listed in the identified Uniprot entries are incorporated herein by reference.

In the preceding description, for purposes of explanation, numerous details are set forth in order to provide a thorough understanding of the examples. However, it will be apparent to one skilled in the art that these specific details are not required. Accordingly, what has been described is merely illustrative of the application of the described examples and numerous modifications and variations are possible in light of the above teachings.

Since the above description provides examples, it will be appreciated that modifications and variations can be effected to the particular examples by those of skill in the art. Accordingly, the scope of the claims should not be limited by the particular examples set forth herein, but should be construed in a manner consistent with the specification as a whole.

## Claims

1. An epidermal growth factor receptor (EGFR) pathway activator for use in the treatment of a disease or disorder **characterized by** satellite cells having an inability, or a reduced ability, to assemble a functional dystrophin-associated glycoprotein complex (DGC) that results in an inability, or reduced ability, to establish cell polarity, wherein the EGFR pathway activator is formulated for administration in an amount sufficient to stimulate asymmetric division of at least some satellite stem cells,
wherein the disease or disorder is muscular dystrophy and the satellite stem cells are **characterized by**: expression of paired box 7 (PAX7) protein and EGFR protein, and by a lack of expression of myogenic factor 5 (Myf5) protein and MyoD protein, and
wherein the EGFR pathway activator is a direct activator of the EGFR pathway.

2. The EGFR pathway activator for use according to claim 1, wherein the muscular dystrophy is: Duchenne muscular dystrophy, a dystroglycanopathy, a congenital muscular dystrophy, or a limb-girdle muscular dystrophy.

3. The EGFR pathway activator for use according to claim 1, wherein the satellite stem cells are dystrophin-deficient satellite stem cells.

4. The EGFR pathway activator for use according to any one of claims 1-3, wherein:
the EGFR pathway activator is formulated for administration in an amount that results in at least a 20% increase in levels of phosphorylated EGFR in the satellite stem cells;
the EGFR pathway activator is formulated for administration in an amount that activates Aurka kinase A and induces apicobasal polarity in the at least some satellite stem cells;
the EGFR pathway activator is formulated for administration in an amount that increases the proportion of asymmetric divisions in the satellite stem cells to a level that is at least 50% of the proportion of asymmetric divisions in a population of normal satellite stem cells; or
any combination thereof.

5. The EGFR pathway activator for use according to any preceding claim, wherein the direct EGFR pathway activator is:
epidermal growth factor (EGF),
TGF-alpha,
amphiregulin,
heparin-binding EGF-like growth factor (HB-EGF),
betacellulin,
epiregulin,
an anti-EGFR antibody or an antigen-binding fragment thereof, or
a nanoparticle conjugate thereof.

6. The EGFR pathway activator for use according to claim 5, wherein the direct EGFR pathway activator is an anti-EGFR antibody that binds to an EGF-binding domain on the EGFR.

7. The EGFR pathway activator for use according to claim 5, wherein the direct EGFR pathway activator is an antigen-binding fragment of the anti-EGFR antibody that is a cell-penetrating nanobody that binds an intracellular kinase domain of EGFR.

8. The EGFR pathway activator for use according to any one of claims 1-7, wherein said use improves or rescues satellite cell function; improves or restores the proportion of asymmetric divisions of the satellite stem cells; ameliorates the severity of one or more symptoms of the disease or disorder; or any combination thereof.

## Patentansprüche

1. Weg-Aktivator des epidermalen Wachstumsfaktor-Rezeptors (EGFR) zur Verwendung bei der Behandlung einer Krankheit oder Störung, die durch Satellitenzellen gekennzeichnet ist, die eine Unfähigkeit oder eine verringerte Fähigkeit aufweisen, einen funktionellen Dystrophin-assoziierten Glykoproteinkomplex (DGC) zu bilden, was zu einer Unfähigkeit oder einer verringerten Fähigkeit führt, Zellpolarität zu etablieren, wobei der EGFR-Weg-Aktivator zur Verabreichung in einer Menge formuliert ist, die ausreicht, um die asymmetrische Teilung von mindestens einigen Satellitenstammzellen zu stimulieren,
wobei die Krankheit oder Störung Muskeldystrophie ist und die Satellitenstammzellen **gekennzeichnet sind durch**: Expression des Paired Box 7 (PAX7)-Proteins und des EGFR-Proteins und durch eine mangelnde Expression des Myogenic Factor 5 (Myf5)-Proteins und des MyoD-Proteins, und
wobei der EGFR-Weg-Aktivator ein direkter EGFR-Weg-Aktivator ist.

2. EGFR-Weg-Aktivator zur Verwendung nach Anspruch 1, wobei die Muskeldystrophie ist: Duchenne-Muskeldystrophie, eine Dystroglykanopathie, eine kongenitale Muskeldystrophie oder eine Gliedergürtel-Muskeldystrophie.

3. EGFR-Weg-Aktivator zur Verwendung nach Anspruch 1, wobei die Satellitenstammzellen dystrophinarme Satellitenstammzellen sind.

4. EGFR-Weg-Aktivator zur Verwendung nach einem der Ansprüche 1-3, wobei:
der EGFR-Weg-Aktivator zur Verabreichung in einer Menge formuliert ist, die zu einer mindestens 20%igen Erhöhung der Konzentrationen von phosphoryliertem EGFR in den Satellitenstammzellen führt;
der EGFR-Weg-Aktivator zur Verabreichung in einer Menge formuliert ist, die die Aurka-Kinase A aktiviert und die apikobasale Polarität in den mindestens einigen Satellitenstammzellen induziert;
der EGFR-Weg-Aktivator zur Verabreichung in einer Menge formuliert ist, die den Anteil der asymmetrischen Teilungen in den Satellitenstammzellen auf eine Konzentration erhöht, die mindestens 50% des Anteils der asymmetrischen Teilungen in einer Population normaler Satellitenstammzellen beträgt; oder
eine beliebige Kombination davon.

5. EGFR-Weg-Aktivator zur Verwendung nach einem der vorhergehenden Ansprüche, wobei der direkte EGFR-Weg-Aktivator ist:
epidermaler Wachstumsfaktor (EGF),
TGF-alpha,
Amphiregulin,
Heparin-bindender EGF-ähnlicher Wachstumsfaktor (HB-EGF),
Betacellulin,
Epiregulin,
ein Anti-EGFR-Antikörper oder ein Antigen-bindendes Fragment davon, oder
ein Nanopartikel-Konjugat davon.

6. EGFR-Weg-Aktivator zur Verwendung nach Anspruch 5, wobei der direkte EGFR-Weg-Aktivator ein Anti-EGFR-Antikörper ist, der an eine EGF-Bindungsdomäne auf dem EGFR bindet.

7. EGFR-Weg-Aktivator zur Verwendung nach Anspruch 5, wobei der direkte EGFR-Weg-Aktivator ein Antigen-bindendes Fragment des Anti-EGFR-Antikörpers ist, das ein zelldurchdringender Nanokörper ist, der eine intrazelluläre Kinasedomäne des EGFR bindet.

8. EGFR-Weg-Aktivator zur Verwendung nach einem der Ansprüche 1 bis 7, wobei die Verwendung die Funktion der Satellitenzellen verbessert oder rettet; den Anteil der asymmetrischen Teilungen der Satellitenstammzellen verbessert oder wiederherstellt; den Schweregrad eines oder mehrerer Symptome der Krankheit oder Störung verbessert; oder irgendeine Kombination davon.

## Revendications

1. Activateur de la voie du récepteur du facteur de croissance épidermique (EGFR) pour une utilisation dans le traitement d'une maladie ou d'un trouble **caractérisé par** une incapacité, ou une capacité réduite, des cellules satellites à assembler un complexe glycoprotéique associé à la dystrophine (DGC) fonctionnel, ayant pour résultat une incapacité ou une capacité réduite à établir la polarité cellulaire, l'activateur de la voie de l'EGFR étant formulé pour une administration en une quantité suffisante pour stimuler la division asymétrique d'au moins certaines cellules souches satellites,
la maladie ou le trouble étant la dystrophie musculaire, et les cellules souches satellites étant **caractérisées par** : l'expression de la protéine PAX7 (paired box 7) et de la protéine EGFR, et par un manque d'expression de la protéine du facteur myogénique 5 (Myf5) et de la protéine MyoD, et
l'activateur de la voie de l'EGFR étant un activateur direct de la voie de l'EGFR.

2. Activateur de la voie de l'EGFR pour une utilisation selon la revendication 1, la dystrophie musculaire étant : la dystrophie musculaire de Duchenne, une dystroglycanopathie, une dystrophie musculaire congénitale ou une dystrophie des ceintures.

3. Activateur de la voie de l'EGFR pour une utilisation selon la revendication 1, les cellules souches satellites étant des cellules souches satellites déficientes en dystrophine.

4. Activateur de la voie de l'EGFR pour une utilisation selon l'une quelconque des revendications 1 à 3,
l'activateur de la voie de l'EGFR étant formulé pour une administration en une quantité ayant pour résultat une augmentation d'au moins 20 % des taux d'EGFR phosphorylé dans les cellules souches satellites ;
l'activateur de la voie de l'EGFR étant formulé pour une administration en une quantité qui active la kinase A Aurka et induit la polarité apicobasale dans au moins certaines cellules souches satellites ;
l'activateur de la voie de l'EGFR étant formulé pour une administration en une quantité qui augmente la proportion de divisions asymétriques dans les cellules souches satellites à un taux qui représente au moins 50 % de la proportion de divisions asymétriques dans une population de cellules souches satellites normales ; ou
l'une quelconque de leurs combinaisons.

5. Activateur de la voie de l'EGFR pour une utilisation selon l'une quelconque des revendications précédentes, l'activateur direct de la voie de l'EGFR étant :
le facteur de croissance épidermique (EGF),
le TGF-alpha,
l'amphiréguline,
le facteur de croissance de type EGF se liant à l'héparine (HB-EGF),
la bêtacelluline,
l'épiréguline,
un anticorps anti-EGFR ou un de ses fragments se liant à l'antigène, ou un de ses conjugués de nanoparticules.

6. Activateur de la voie de l'EGFR pour une utilisation selon la revendication 5, l'activateur direct de la voie de l'EGFR étant un anticorps anti-EGFR qui se lie à un domaine de liaison à l'EGF sur l'EGFR.

7. Activateur de la voie de l'EGFR pour une utilisation selon la revendication 5, l'activateur direct de la voie de l'EGFR étant un fragment se liant à l'antigène de l'anticorps anti-EGFR qui est un nanocorps pénétrant les cellules qui se lie à un domaine de kinase intracellulaire de l'EGFR.

8. Activateur de la voie de l'EGFR pour une utilisation selon l'une quelconque des revendications 1 à 7, ladite utilisation améliorant ou sauvant la fonction des cellules satellites ; améliorant ou restaurant la proportion de divisions asymétriques des cellules souches satellites ; atténuant la gravité d'un ou plusieurs symptômes de la maladie ou du trouble ; ou l'une quelconque de leurs combinaisons.
